(19) Europäisches Patentamt / European Patent Office / Office européen des brevets

(11) **EP 3 030 896 B1**

(12) **FASCICULE DE BREVET EUROPEEN**

(45) Date de publication et mention
de la délivrance du brevet:
**15.07.2020 Bulletin 2020/29**

(51) Int Cl.:
**G01N 33/02** *(2006.01)*    **G01N 33/00** *(2006.01)*

(21) Numéro de dépôt: **14755887.8**

(22) Date de dépôt: **06.08.2014**

(86) Numéro de dépôt international:
**PCT/FR2014/052046**

(87) Numéro de publication internationale:
**WO 2015/019023 (12.02.2015 Gazette 2015/06)**

(54) **COMPOSITIONS DE BIOMASSE DE MICROALGUES RICHES EN PROTEINES DE QUALITE SENSORIELLE OPTIMISEE**

PROTEINREICHE MIKROALGEN-BIOMASSE-ZUSAMMENSETZUNGEN VON OPTIMIERTER SENSORISCHER QUALITÄT

PROTEIN-RICH MICROALGAL BIOMASS COMPOSITIONS OF OPTIMIZED SENSORY QUALITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.08.2013 FR 1357843**

(43) Date de publication de la demande:
**15.06.2016 Bulletin 2016/24**

(73) Titulaire: **Corbion Biotech, Inc.**
**South San Francisco, CA 94080 (US)**

(72) Inventeurs:
• **JEROSCH, Heike**
**59940 Estaires (FR)**
• **DRUON, Amandine**
**59000 Lille (FR)**
• **GUILLEMANT, Marilyne**
**62120 Aire sur la Lys (FR)**
• **PATINIER, Samuel**
**59890 Quesnoy sur Deule (FR)**

(74) Mandataire: **Cabinet Becker et Associés**
**25, rue Louis le Grand**
**75002 Paris (FR)**

(56) Documents cités:
**EP-A1- 2 948 001**

• **MÓNICA FRADIQUE ET AL: "Incorporation of Chlorella vulgaris and Spirulina maxima biomass in pasta products. Part 1: Preparation and evaluation", JOURNAL OF THE SCIENCE OF FOOD AND AGRICULTURE, vol. 90, no. 10, 15 août 2010 (2010-08-15) , pages 1656-1664, XP055027243, ISSN: 0022-5142, DOI: 10.1002/jsfa.3999**
• **SANG-MI SUN ET AL: "Volatile compounds of the green alga", JOURNAL OF APPLIED PHYCOLOGY, KLUWER ACADEMIC PUBLISHERS, DO, vol. 24, no. 5, 27 septembre 2011 (2011-09-27), pages 1003-1013, XP035108730, ISSN: 1573-5176, DOI: 10.1007/S10811-011-9724-X**
• **BECKER ET AL: "Micro-algae as a source of protein", BIOTECHNOLOGY ADVANCES, ELSEVIER PUBLISHING, BARKING, GB, vol. 25, no. 2, 26 janvier 2007 (2007-01-26), pages 207-210, XP005862318, ISSN: 0734-9750, DOI: 10.1016/J.BIOTECHADV.2006.11.002**
• **Jaclyn E R Santos ET AL: "Analysis of Volatile Organic Compounds in Virgin Coconut Oil and their Sensory Attibutes", Philippine Journal of Science, 19 décembre 2011 (2011-12-19), pages 161-171, XP055139768, Extrait de l'Internet: URL:http://philjournalsci.dost.gov.ph/vol1 40no2/pdfs/Analysis of volatile organic compounds in VCO.pdf [extrait le 2014-09-11]**

## Description

La présente invention est relative à une méthode pour évaluer le profil organoleptique de nouvelles compositions de biomasse de microalgues du genre *Chlorella* riche en protéines présentant un profil sensoriel optimisé, ce qui permet de les incorporer dans des formulations alimentaires sans génération d'arômes indésirables.

### *Présentation de l'état de l'art*

Il est bien connu de l'homme du métier que les chlorelles sont une source potentielle de nourriture, car elles sont riches en protéines et autres nutriments essentiels.

Elles renferment notamment 45% de protéines, 20% de matières grasses, 20% de glucides, 5% de fibres et 10% de minéraux et de vitamines.

L'utilisation des biomasses de microalgues (et principalement leurs protéines) comme aliments est de plus en plus considérée pour trouver des sources alternatives à la demande croissante en protéines animales au niveau mondial (comme rapporté par la FAO).

L'Union européenne, depuis des années, souffre par ailleurs d'un déficit structurel en protéines végétales qui s'élève, ces dernières années, à plus de 20 millions de tonnes d'équivalent soja, aujourd'hui importés d'Amérique du Sud.

La production de masse de certaines microalgues riches en protéines est alors envisagée comme une possibilité de combler ce « déficit en protéines ».

Des analyses exhaustives et des études nutritionnelles ont montré que ces protéines d'algues sont équivalentes aux protéines végétales conventionnelles, voire de meilleure qualité.

Toutefois, en raison des coûts élevés de production ainsi que des difficultés techniques pour intégrer le matériel issu de microalgues dans des préparations alimentaires organoleptiquement acceptables, la diffusion large des protéines de microalgues est encore à ses balbutiements.

Des biomasses de microalgues de différentes espèces présentant un pourcentage élevé en protéines ont été rapportées (voir le tableau 1 de Becker, Biotechnology Advances (2007)25:207-210).

Un certain nombre de demandes de brevet dans l'état de l'art, telle la demande de brevet WO 2010/045368, enseignent quant à elles qu'il est possible d'ajuster les conditions de culture de manière à augmenter encore la teneur en protéines de la biomasse de microalgues.

Cependant, lorsque l'on souhaite fabriquer industriellement des poudres de biomasse de microalgues à partir de leur biomasse, d'importantes difficultés demeurent, non seulement du point de vue technologique, mais également du point de vue du profil sensoriel des compositions produites.

En effet, si des poudres d'algues, par exemple fabriquées avec des algues cultivées photosynthétiquement dans des étangs extérieurs ou par photobioréacteurs, sont disponibles dans le commerce, elles ont une couleur vert foncée (liée à la chlorophylle) et un goût fort, désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines. L'évaluation sensorielle de compositions de biomasses de microalgues des genres *Chlorella* et *Spirulina* dans des pâtes alimentaires est décrite par exemple dans Fradique, J Sci Food Agric 2010; 90: 1656-1664. Quant à Sun, J Appl Phycol (2012) 24:1003-1013, cet art antérieur divulgue l'analyse en composés organiques volatils de l'algue verte *Capsosiphon fulvescens* suivant la méthode d'extraction des huiles essentielles afin de comprendre l'impacte aromatique de cette algue lorsqu'elle est utilisée comme source de protéines dans des produits alimentaires. De plus, EP2948001 A0, document faisant partie de l'art antérieur au sens de l'Article 54(3) CBE, décrit une méthode pour améliorer la production de farines de microalgues suivant des caractéristiques organoleptiques acceptables déterminées par la détermination de la teneur en composés organiques volatils spécifiques.

Par ailleurs, il est connu que certaines espèces d'algues bleues produisent naturellement des molécules chimiques odorantes telles que la géosmine (trans-1,10-diméthyle-trans-9-decalol) ou le MIB (2-méthylisoborneol), générant des odeurs terreuses ou de moisi.

Quant aux chlorelles, le descripteur communément admis dans ce domaine est le goût de « thé vert », un peu semblable à d'autres poudres végétales vertes telles que l'orge vert en poudre ou le blé vert en poudre, goût attribué à sa forte teneur en chlorophylle.

Leur saveur n'est habituellement masquée que lorsqu'elles sont mélangées avec des légumes à saveur forte ou des jus d'agrumes.

Il existe donc toujours un besoin non satisfait de disposer de compositions de biomasse de microalgues du genre *Chlorella* de qualité organoleptique convenable permettant l'utilisation de celles-ci dans des produits alimentaires plus nombreux et diversifiés.

***Résumé de l'invention***

La société Demanderesse a trouvé que l'on pouvait répondre à ce besoin en proposant des compositions de biomasse de microalgues riches en protéines présentant un profil sensoriel optimisé caractérisées par la valeur globale d'arômes de 4 composés organiques volatils choisis parmi 11 composés particuliers.

Ainsi, la présente invention est tout d'abord relative à une méthode pour déterminer la qualité organoleptique d'une composition de biomasse de microalgues riche en protéines comprenant au minimum 50 % de protéines en poids sec de biomasse de microalgues, caractérisée en ce que la méthode comprend la détermination de la teneur en 11 composés organiques volatils, les 11 composés organiques volatils étant le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, (E,E)-2,4-nonadiénal et l'acide hexanoïque, la biomasse de microalgues comprenant plus de 50 % de protéines en poids sec de biomasse et les microalgues étant du genre Chlorella.

De préférence, la teneur de chacun de ces 11 composés organiques volatils est déterminée par SPME / GC, de préférence par SPME / GC-MS.

De préférence, la teneur en chacun de ces 11 composés organiques volatils est déterminée par la surface des pics de chromatographie après SPME / GC correspondant à chacun de ces 11 composés organiques volatils.

De préférence, la teneur en chacun de ces 11 composés organiques volatils, en particulier la surface des pics de chromatographie correspondant aux 11 composés organiques volatiles, est comparée à celle de composition de biomasse de microalgues riche en protéines de référence pour laquelle ou lesquelles les qualités organoleptiques sont définies, notamment comme inacceptable ou acceptable.

La présente invention est enfin relative à la sélection de 4 des 11 composés organiques volatils présentant un faible seuil olfactif (c'est-à-dire impactant de façon majeure l'odeur globale ressentie par les membres du panel sensoriel), pour bâtir un modèle simplifié permettant de donner une valeur d'arôme globale aux compositions de biomasse de microalgues riches en protéines. Ainsi, elle est relative à une méthode pour déterminer la qualité organoleptique d'une composition de biomasse de microalgues riche en protéines comprenant au minimum 50 % de protéines en poids sec de biomasse de microalgues, caractérisée en ce que la méthode comprend la détermination de la teneur en 4 composés organiques volatils, ces 4 composés organiques étant le 3,5-octadiène-2-ol, le 1-octène-3-ol, du 3,5-octadiène-2-one, et le (E,E)-2,4-nonadiénal et en ce que calcule une somme des valeurs d'arômes individuelles (= concentration du composé / son seuil olfactif) du 3,5-octadiène-2-ol, du 1-octène-3-ol, du 3,5-octadiène-2-one, et du (E,E)-2,4-nonadiénal, la biomasse de microalgues comprenant plus de 50 % de protéines en poids sec de biomasse et les microalgues étant du genre Chlorella.

Les compositions de biomasse de microalgues riches en protéines conformes à l'invention présentent donc un profil sensoriel optimisé lorsque leur valeur d'arome globale est faible, de préférence comprise entre 0 et 40 % par rapport à celle d'une composition de farine de microalgues de référence organoleptique inacceptable.

La présente invention est également relative à une méthode de sélection de compositions de biomasse de microalgues riches en protéines présentant un profil organoleptique acceptable, caractérisée en ce que la qualité organoleptique est déterminée par la méthode telle que décrite ci-dessus et que la composition est sélectionnée lorsque la valeur d'arôme globale calculée par la méthode telle que décrite ci-dessus est comprise entre 0 et 40 % par rapport à celle d'une composition de biomasse de microalgues de référence organoleptique inacceptable. De préférence, la biomasse de microalgues comprend plus de 50 % de protéines en poids sec de biomasse et en ce que les microalgues sont du genre Chlorella.

De préférence, les microalgues sont du genre Chlorella, en particulier choisies dans le groupe constitué de *Chlorella vulgaris, Chlorella sorokiniana* et *Chlorella protothecoides,* et plus particulièrement *Chlorella protothecoides.*

***Description détaillée de l'invention***

Au sens de l'invention, une composition de biomasse de microalgues riche en protéines présente un « profil sensoriel optimisé » ou une « qualité organoleptique optimisée », lorsque son évaluation par un panel sensoriel conclut à l'absence de défauts qui altèrent la qualité organoleptique desdites formulations alimentaires contenant ces compositions de biomasse de microalgues.

Par « qualité organoleptique » est entendue la propriété d'un aliment en termes de couleur et d'odeur.

Ces défauts sont associés à la présence de molécules spécifiques odorantes et/ou aromatiques indésirables qui se caractérisent par un seuil de perception correspondant à la valeur minimale du stimulus sensoriel nécessaire à l'éveil d'une sensation.

Le « profil sensoriel optimisé » ou « qualité organoleptique optimisé » est alors traduit par un panel sensoriel par l'obtention des meilleurs scores sur une échelle d'évaluation des 2 critères sensoriels (couleur et odeurs).

Par « environ » est entendue la valeur plus ou moins 10 % de celle-ci, de préférence plus ou moins 5 % de celle-ci. Par exemple, « environ 100 » signifie entre 90 et 110, de préférence entre 95 et 105.

Par « composition de biomasse de microalgues » est entendu une composition comprenant au minimum 50, 60, 70, 80 ou 90 % en poids sec de biomasse de microalgues. Cependant, d'autres ingrédients peuvent facultativement être compris dans cette composition.

Par « riche en protéine » est entendu une teneur en protéines dans la biomasse de plus de 50 % en poids sec, de préférence de plus de 55 %, plus préférentiellement encore de plus de 60, 65 et 70 % en poids sec de biomasse.

Au sens de la présente invention, le terme « biomasse de microalgues » doit être compris dans son interprétation la plus large et comme désignant par exemple, une composition comprenant une pluralité de particules de biomasse de microalgues. La biomasse de microalgues est dérivée de cellules de microalgues qui sont entières.

Un certain nombre de documents de l'état de l'art, comme la demande de brevet internationale WO 2010/045368, décrivent des méthodes de fabrication et d'utilisation en alimentation de la biomasse de microalgues de *Chlorella* riche en protéines.

Les microalgues dont il est question dans la présente invention sont donc des microalgues du genre *Chlorella,* plus particulièrement *Chlorella protothecoides,* plus particulièrement encore des *Chlorella* privées de pigments chlorophylliens, par toute méthode connue en soi de l'homme du métier (soit par le fait que la culture est réalisée à l'obscurité, soit parce que la souche a été mutée de manière à ne plus produire ces pigments).

Notamment, les microalgues peuvent être choisies, de manière non-exhaustive, parmi les *Chlorella protothecoides, Chlorella kessleri, Chlorella minutissima, Chlorella sp., Chlorella sorokiniama, Chlorella luteoviridis, Chlorella vulgaris, Chlorella reisiglii, Chlorella ellipsoidea, Chlorella saccarophila, Parachlorella kessleri, Parachlorella beijerinkii, Prototheca stagnora* et *Prototheca moriformis.* Ainsi, dans un mode de réalisation tout particulier, la composition de biomasse de microalgues est une composition de biomasse de *Chlorella,* et en particulier de *Chlorella protothecoides.*

Le procédé fermentaire décrit dans cette demande de brevet WO 2010/045368 permet ainsi la production d'un certain nombre de compositions de biomasse de microalgues de qualité sensorielle variable.

La méthode telle que décrite dans le présent document permet donc de sélectionner les compositions de biomasse de microalgues riches en protéines présentant un profil organoleptique acceptable, notamment pour les applications alimentaires, sans pour cela devoir organiser des évaluations organoleptiques par un panel de personnes.

### 1. Définition du profil sensoriel par la détection de 11 composés organiques volatils

La société Demanderesse a découvert que le profil sensoriel d'une composition de biomasse de microalgues riche en protéines peut être défini par la nature et le seuil de détection de molécules spécifiques odorantes, en particulier de composés organiques volatils particuliers.

En effet, elle a identifié un ensemble de 11 composés organiques volatils dont la teneur dans une composition de biomasse de microalgues riche en protéines permet de déterminer la qualité organoleptique de celle-ci.

Ces 11 composés organiques volatils sont les suivants : le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, le (E,E)-2,4-nonadiénal et l'acide hexanoïque.

Ainsi, la présente invention est relative à une méthode pour déterminer la qualité organoleptique d'une composition de biomasse de microalgues riche en protéines comprenant la détermination de la teneur en chacun de ces 11 composés organiques volatils, les 11 composés organiques volatils étant le pentanal, l'hexanal, le 1-octène-3-ol, le 2-pentylfuran, l'octanal, le 3,5-octadiène-2-ol (ou 3-octène-2-one), le 3,5-octadiène-2-one, le nonanal, le 2-nonénal, (E,E)-2,4-nonadiénal et l'acide hexanoïque.

La méthode n'exclut pas la détermination de la teneur d'autres composés organiques volatils. Cependant, les 11 composés organiques volatils sont suffisants pour déterminer la qualité organoleptique d'une composition de biomasse de microalgues riche en protéines.

De préférence, ces composés organiques volatils sont prélevés par micro extraction en phase solide (SPME) et analysés par chromatographie en phase gazeuse GC, en particulier par GC-MS (chromatographie en phase gazeuse couplée à la spectrométrie de masse).

La fraction volatile est extraite de l'échantillon de la composition de biomasse de microalgues riche en protéines en chauffant celle-ci pendant une durée suffisante en présence d'une fibre de SPME.

La fibre peut être par exemple choisie, de manière non-exhaustive, parmi le groupe consistant en carboxen et polydiméthylsiloxane (CAR/PDMS), divinylbenzène, Carboxen et polydiméthylsiloxane (DVB/CAR/PDMS), un alliage de métal et de polydiméthylsiloxane (PDMS), une fibre Carbopack-Z® (carbone noir graphitisé), polyacrylate, Carbowax® polyéthylèneglycol (PEG), et PDMS / DVB.

De préférence, on utilise une fibre DVB/CAR/PDMS (df 50/30 $\mu$m).

La société Demanderesse recommande d'utiliser ici une technique d'extraction en voie humide (suspension aqueuse) entre 40 et 70°C, de préférence entre 50 et 65°C, en particulier environ 60 °C pendant au moins 10 minutes, de préférence au moins 15 minutes et par exemple entre 15 minutes et 1 heure.

De préférence, cette étape d'extraction est faite dans un récipient scellé. Une quantité suffisante d'échantillon doit être utilisée, par exemple au moins 1 g, notamment entre 1 et 10 g et en particulier environ 2 g.

Ces 2 g sont alors mis en suspension dans 7 ml d'eau contenant 1 g de CaCl2, 200 $\mu$l de HCl et 2,32 $\mu$g d'hexanal-d12 (étalon interne), introduit dans un flacon SPME (20 ml) scellé.

Les composés organiques volatils sont ensuite désorbés, à une température compatible avec le type de fibre SPME utilisée, par exemple entre 220 et 250 °C pour la fibre utilisée dans nos essais, plus précisément à 230°C, et injectés dans le système d'analyse.

De préférence, l'analyse est faite par chromatographie en phase gazeuse GC, en particulier par GC-MS.

Plusieurs dispositifs de GC/MS sont disponibles commercialement, par exemple le spectromètre GC/Masse Clarus (PerkinElmer, USA), le chromatographe en phase gazeuse Hewlett Packard 6890 (Hewlett Packard, USA) et le chromatographe en phase gazeuse Aglient 6890N couplé détecteur sélectif de masse Aglient 5973.

Les méthodes d'ionisation qui peuvent être utilisées en GC/MS sont par exemple la spectroscopie de masse avec ionisation en mode impact électronique (EI), en mode impact chimique (CI) l'ionisation par électronébulisateur, la décharge luminescente, la désorption par champs (FD), etc.

Les volatils extraits sont ici plus précisément désorbés dans l'injecteur du système GC-MS TSQ de Thermo Scientific, puis séparés sur une colonne CPwax52 (60m * 0.25 mm, 0.25 $\mu$m) avec le gaz Hélium à 1.5 ml/min.

Le programme de température est :

-   50°C isotherme pendant 3 min, puis
-   programmation à 5°C/minute jusque 230°C,
-   puis isotherme pendant 20 min.

La détection est faite par spectrométrie de masse (MS) en mode impact électronique (EI) et les composés sont identifiés par comparaison avec les spectres EI de la bibliothèque NIST.

Ainsi, la hauteur ou la surface du pic de chromatographie correspondant au composé organique volatil est corrélée à la quantité dudit composé.

Par « surface du pic » est entendue la surface d'un ion spécifique sous la courbe dans le chromatogramme SPME-GC/MS.

De préférence, la teneur en un des 11 composés organiques volatils est déterminée par la surface du pic de l'ion spécifique du chromatogramme SPME-GC/MS correspondant à ce composé organique volatil.

La teneur en composés organiques volatils est déterminée, notamment en comparaison à celle de produit de référence.

Ainsi, globalement, une faible teneur totale en les 11 composés organiques volatils est associée à une qualité organoleptique optimisée. A contrario, une teneur totale plus élevée en les 11 composés organiques volatils est associée à une qualité organoleptique moyenne, voire mauvaise ou inacceptable.

Par exemple, la teneur totale d'une composition d'une qualité organoleptique acceptable est faible lorsqu'elle est au minimum deux fois inférieure à celle d'une composition d'une qualité organoleptique inacceptable, par exemple au minimum 2, 3, ou 4 fois inférieure, et dans un mode de réalisation le plus exigeant, au minimum 10 fois inférieure.

### *2. Modèle simplifié basé sur quatre composés organiques volatils impactant l'odeur globale*

Dans ce mode de réalisation préféré, la société Demanderesse a trouvé que l'on peut avantageusement établir une valeur globale d'arôme pour les compositions de biomasse de microalgues riches en protéines présentant un profil sensoriel optimisé, valeur globale basée sur 4 composés organiques volatils choisis parmi les 11 composés organiques identifiés ci-dessus.

Ces composés organiques volatils sont sélectionnés sur leur critère de faible seuil olfactif. La valeur globale d'arome est alors établie selon la relation :

Valeur globale d'arôme = $\Sigma$ des valeurs arômes individuels du 3,5-octadiène-2-ol (ou 3-octène-2-one, option ne faisant pas partie de l'invention), du 1-octène-3-ol, du 3,5-octadiène-2-one, et du (E,E)-2,4-nonadiénal.

$$VA\ total=\Sigma\ [\ VA(3,5\text{-octadiène-2-ol}),\ VA(1\text{-octène-3-ol}),\ VA(3,5\text{-octadiène-2-one}), et\ VA[(E,E)\text{-2,4-nonadiénal}],$$

avec *VA = Concentration du composé x/ seuil olfactif du composé x)*

Comme il sera exemplifié ci-après, les compositions de biomasse de microalgues riches en protéines qui présentent une valeur globale d'arôme faible, comprise entre 0 et 40 % par rapport à celle d'une composition de farine de microalgues de référence organoleptique inacceptable, sont assurées de présenter un profil sensoriel optimisé.

L'invention sera mieux comprise à l'aide des exemples qui suivent.

## EXEMPLES

### Exemple 1. Définition du test sensoriel

La perception de la composition de biomasse de microalgues riche en protéines est déterminée par solubilisation dans de l'eau, milieu neutre par excellence.

Un panel sensoriel a donc été formé pour évaluer, selon la méthodologie présentée ci-avant, les propriétés sensorielles de différents lots de biomasse de microalgues riches en protéines, préparées selon l'enseignement de la demande de brevet WO 2010/045368.

18 lots de biomasse de microalgues ont été testés : lot 11, lot 12, lot 14, lot 33, lot 34, lot 42, lot 43, lot 44, lot 54, lot 81, lot 82, lot 83, lot 84, lot 85, lot 92, lot 93, lot 111, lot 112.

On présente le résultat d'une telle caractérisation des lots sur le descripteur très caractéristique d'odeur de « peinture ».

### Traitement des données

Les analyses ont été réalisées à l'aide du logiciel R (en vente libre) :

R version 2.14.1 (2011-12-22)

Copyright (C) 2011 The R Foundation for Statistical Computing

ISBN 3-900051-07-0

Platform: i386-pc-mingw32/i386 (32-bit)

Le logiciel est un environnement de travail qui nécessite le chargement de modules contenant les fonctions de calcul.

Les modules utilisés pour le traitement de données de profil sont les suivants :

- Pour l'ANOVA : Package car version 2.0-12

- Pour la régression linéaire : Package stats version 2.14.1

L'ANOVA montre des résultats significativement différents d'un produit à l'autre :

Tableau Anova (tests de Type III)

Réponse: Donnée [,peinture]

|  | SCEM | ddl | valeur de F | **Pr(>F)** |
|---|---|---|---|---|
| (moyenne) | 327,94 | 1 | 234,1068 | < 2,2e-16 |
| **Composition** | 684,40 | 17 | 28,7393 | **< 2,2e-16** |
| Panéliste | 118,84 | 19 | 4,4649 | 7,097e-09 |
| Résidus | 410,44 | 293 | | |

Voici les moyennes obtenues par produits :

| Compositions | moyenne | écart type | répétition |
|---|---|---|---|
| Lot 092 | 0,00 | 0,00 | 8 |
| Lot 111 | 0,00 | 0,00 | 9 |

(suite)

| Compositions | moyenne | écart type | répétition |
|---|---|---|---|
| Lot 12 | 0,10 | 0,10 | 10 |
| Lot 112 | 0,28 | 0,12 | 36 |
| Lot 43 | 0,29 | 0,17 | 21 |
| Lot 44 | 0,33 | 0,26 | 12 |
| Lot 33 | 0,36 | 0,36 | 11 |
| Lot 11 | 0,40 | 0,40 | 10 |
| Lot 81 | 0,96 | 0,25 | 23 |
| Lot 14 | 1,00 | 0,50 | 9 |
| Lot 82 | 1,50 | 0,34 | 24 |
| Lot 34 | 1,64 | 0,38 | 22 |
| Lot 42 | 1,96 | 0,25 | 49 |
| Lot 93 | 2,67 | 0,58 | 9 |
| Lot 84 | 3,23 | 0,36 | 22 |
| Lot 54 | 4,09 | 0,41 | 11 |

| Lot 83 | 4,24 | 0,16 | 34 |
|---|---|---|---|
| Lot 85 | 4,60 | 0,22 | 10 |

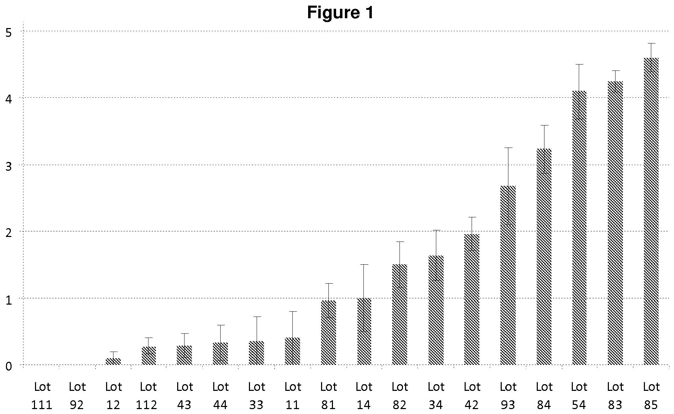

La **Figure 1** présente le classement des différents lots au regard de la note attribuée par les panélistes sur ce critère « peinture ».

Le classement est alors le suivant, par ordre de note « peinture » croissante :

Lot 92 > lot 111 > lot 12 > lot 112 > lot 43 > lot 44 > lot 33 > lot 11 > lot 81 > lot 14 > lot 82 > lot 34 > lot 42 > lot 93 > lot 84 > lot 54 > lot 83 > lot 85.

Le lot 92 est alors défini comme le Témoin de qualité organoleptique acceptable pour ce descripteur peinture.

Le lot 85 est quant à lui défini comme le Témoin de qualité organoleptique inacceptable pour ce descripteur peinture.

Cette classification organoleptique désormais établie, il est possible, efficacement selon l'invention, d'analyser le profil en SPME / GC-MS de ces échantillons afin d'identifier les cibles moléculaires de référence qui permettront de définir la qualité des compositions fabriquées.

**Exemple 2. Identification des composés organiques volatils (VOC) par SPME / GC-MS liés aux classifications organoleptiques inacceptables « odeur peinture »**

Pour réaliser l'analyse en SPME / GC-MS des 18 différents lots de compositions de biomasse de microalgues, on procède comme indiqué ci-dessus en suspension aqueuse.

***Analyse des composés volatils sur produits en suspension aqueuse***

Les composés volatils ont été analysés en suspension aqueuse afin de réduire l'effet matrice, et un étalon interne a été introduit.

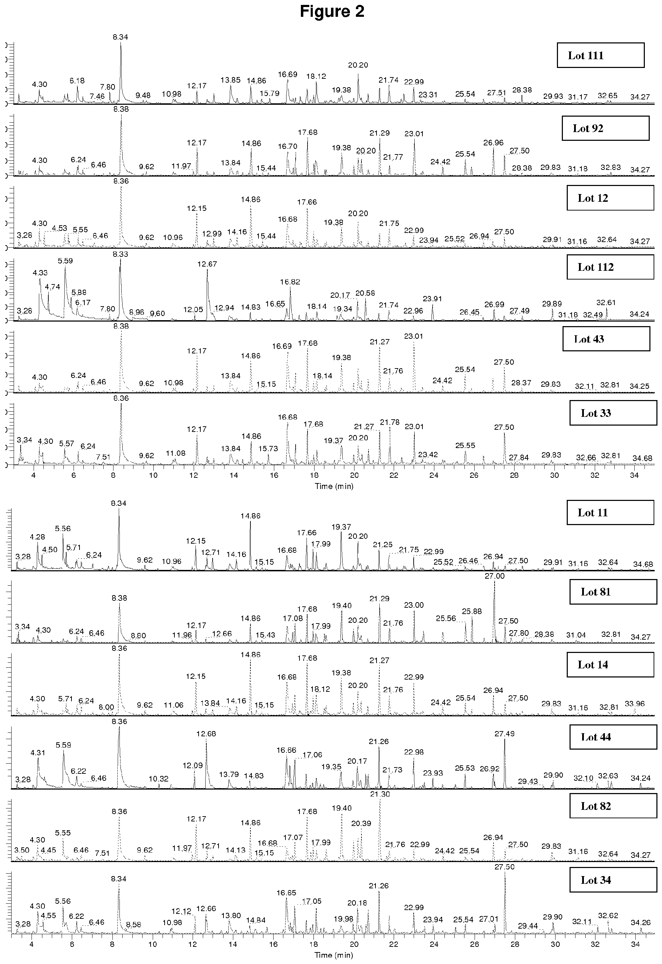

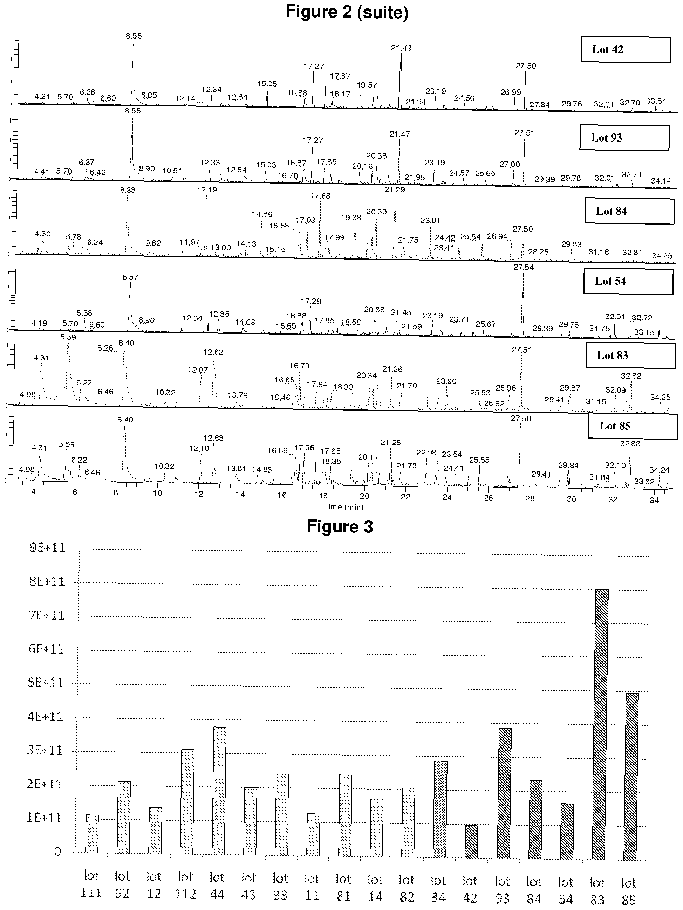

Visuellement, comme le montre la **Figure 2**, les chromatogrammes GC-MS restent très complexes, avec un très grand nombre de composés.

La première approche consiste à comparer les profils chromatographiques, à intégrer la totalité des pics entre 3,2 à 35,0 min (TIC, « total ion current ») et vérifier si ces résultats « bruts » permettent de faire le lien avec le classement sensoriel.

La comparaison des profils chromatographiques et l'intégration de la totalité des pics entre 3,2 à 35,0 min (TIC, « total ion current ») - voir **Figure 3** - ne permettent pas de faire le lien avec le classement sensoriel.

A cause de la grande complexité des chromatogrammes, il est difficile de distinguer visuellement les produits acceptables des inacceptables.

L'intégration des surfaces des chromatogrammes ne permet pas non plus de trancher nettement entre échantillons acceptables et inacceptables.

De plus, cette approche des données brutes ne permet pas de savoir quel ou quels composé(s) volatil(s) est (sont) responsable(s) des off-notes ou goûts ou odeurs indésirables, ni de suivre spécifiquement leur apparition, ni d'avoir des renseignements sur leur voie de formation.

Une seconde approche a consisté à compléter la liste des composés organiques volatils du modèle précédent en listant les composés volatils identifiés sur les chromatogrammes SPME / GC-MS qui semblent accompagner les classifications organoleptiques.

L'analyse GC-MS-Olfactométrie de six échantillons avait fait ressortir certains composés volatils, majoritairement des aldéhydes issus de la dégradation de la fraction lipidique des compositions de biomasse de microalgues riches en protéines, qui seraient responsables des off-notes ou goûts ou odeurs indésirables.

Dans cette deuxième approche, il a alors été décidé de suivre certaines de ces molécules sélectionnées par GC-MS-Olfactométrie et par GC-MS des différents produits.

Pour sélectionner les composés organiques volatils représentatifs, on réalise une série d'analyses de la variance pour ne garder que les composés organiques volatils qui diffèrent effectivement d'une composition à l'autre compte tenue de la variabilité de la mesure SPME - GC/MS.

Le modèle est le suivant : *Composé organique volatil ~ Composition* ; on ne conserve que les composés pour lesquels la probabilité critique associée au test de Fisher est inférieure à 0,05.

On présente ici 2 exemples d'ANOVA sur les composés 2-nonénal et 3.5-octadiène-2-one-2 :

2-nonénal

Tableau Anova (tests de Type III)

|  | SCEM ddl | valeur de F | **Pr(>F)** |
|---|---|---|---|
| (moyenne) | 292,13 1 | 72,8516 | 3,51e-06 |
| **Composition** | 664,79 17 | 9,7522 | **0,0002394** |
| Résidus | 44,11 11 | | |

3,5-octadiène-2-one (pic 2)

Tableau Anova (tests de Type III)

|  | SCEM ddl | valeur de F | **Pr(>F)** |
|---|---|---|---|
| (moyenne) | 56344 1 | 20,0633 | 0,0009326 |
| **Composition** | 72570 17 | 1,5201 | **0,2424099** |
| Résidus | 30891 11 | | |

Il apparait sur le premier composé organique volatil (le 2-nonénal) que l'effet composition est significatif (probabilité critique <0,00025), ce qui signifie qu'il y a une différence significative entre les produits compte tenu de la variabilité de la mesure.

Sur le 2ème composé (3,5-octadiène-2-one, pic 2), l'effet composition n'est pas significatif (probabilité critique > 0,05). On gardera donc pour l'étude le 2-nonénal mais pas le 3,5-octadiène-2-one.

Après cette première sélection de composés volatils, des modèles de régressions linéaires sont établis : il s'agit d'expliquer la variable « peinture » par chaque composé un à un.

On construit donc autant de modèle qu'il y a de composés. Le modèle est le suivant : *Peinture ~ Composé.*

Afin de sélectionner la liste finale de composés identifiés comme responsables des classifications organoleptiques inacceptables (off-notes) observées, on ne gardera que les composés pour lesquels la probabilité critique associée au test de Student est inférieure à 0,05 (test de nullité du coefficient de régression linéaire).

Le $R^2$ associé au modèle est un indicateur pour quantifier le pourcentage de variabilité expliqué par le composé. Il peut ne pas être très élevé mais être significatif, c'est pourquoi on choisit de sélectionner les composés en fonction de la probabilité critique (afin de ne pas négliger un composé qui influe peu mais significativement sur l'odeur peinture décrite par le panel).

Coefficients:

|  | Estimateur | Std valeur | valeur de t | Pr(>\|t\|) |
|---|---|---|---|---|
| *(ordonnée à l'origine)* | -0,669037 | 0,138335 | -4,836 | 0,000182 |
| **Hexanal** | 0,019196 | 0,002593 | 7,404 | **1,49e-06** |

Erreur standard résiduelle: 0,4444 on 16 degrés de liberté
R$^2$ Multiple: 0,7741, R$^2$ ajusté: 0,76
*Statistique* F: 54,82 sur 1 et 16 degrés de liberté, probabilité critique: 1,491e-06

Coefficients:

|  | Estimateur | Std valeur | valeur de t | Pr(>\|t\|) |
|---|---|---|---|---|
| *(ordonnée à l'origine)* | -0,45338 | 0,24852 | -1,824 | 0,0868 |
| **3,5-octadiène-2-one (pic 1)** | 0,04346 | 0,01614 | 2,693 | **0,0160** |

Erreur standard résiduelle: 0,7756 on 16 degrès de liberté
R$^2$ Multiple: 0,3119, R$^2$ ajusté: 0,2689
*Statistique* F: 7,252 sur 1 et 16 degrés de liberté, probabilité critique: 0,016

Pour ces 2 composés, l'hexanal et le 3,5-octadiène-2-one (pic 1), la probabilité critique est inférieure à 0,05, donc ils sont corrélés à l'odeur de peinture décrite par le panel.

Les 11 composés de l'étude se trouvent être bien corrélés au descripteur « peinture ».

Ces 11 molécules sélectionnées sont listées dans le tableau ci-dessous :

|  | Molécule | Temps de rétention (min) | Odeur | Seuil olfactif (PPb) | Ion spécifique m/z |
|---|---|---|---|---|---|
| 1 | Pentanal | 6,24 | Verte | 18* | 44 |
| 2 | Hexanal | 8,24 | Herbe coupée, pomme verte | 4,5 | 82 |
| 3 | 1-octène-3-ol | 17,99 | Champignon-solvant (peinture), champignon-encre | 1 / 0,05* | 57 |
| 4 | 2-pentylfurane | 12,10 | florale | 6 | 81 |
| 5 | Octanal | 13,82 | Floral-agrume | 0,7 | 84 |
| 6 | 3,5-octadiène-2-ol ou 3-octène-2-one | 17,03 | Floral-zeste | 0,1** | 111 |
| 7 | 3,5-octadiène-2-one (2 pics) | 19,96+ 21,24 | florale | 0,1** | 95 |
| 8 | Nonanal | 16,68 | Floral-verte, florale | 1 | 57 |
| 9 | 2-Nonénal | 20,37 | Végétal, huile | 0,08 | 83 |
| 10 | (E,E)-2,4-nonadiénal | 24,42 | Huileuse-oxydée | 0,09 | 81 |
| 11 | Acide hexanoïque | 27,51 | Fromage, rance | 3000 | 60 |

* seuil olfactif selon H. Jelen, Journal of Chromatographic Science, vol. 44, august 2006
** valeur estimée
Sans autre indication, le seuil olfactif est repris de www.leffingwell.com/odorthre.htm

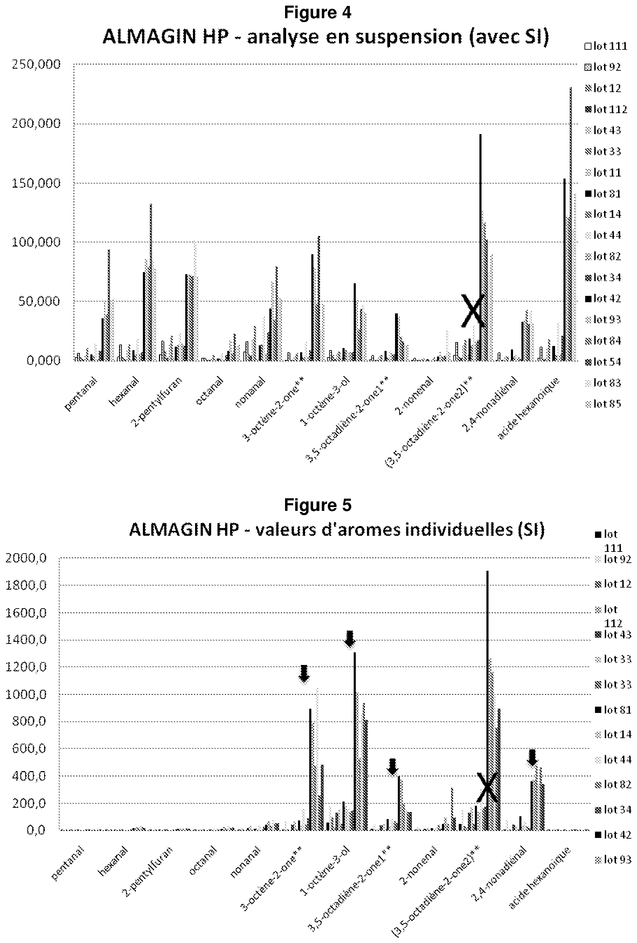

Comme le montre la **Figure 4**, les produits inacceptables semblent bien plus chargés en ces 11 composés volatils que les échantillons acceptables.

L'analyse statistique confirme que toutes les 11 molécules sont significatives (excepté le deuxième pic du 3,5-octadiène-2-one à 21,24 min).

En conclusion, le suivi de ces 11 molécules (pentanal, hexanal, 1-octène-3-ol, 2-pentylfurane, octanal, 3,5-octadiène-2-ol ou 3-octène-2-one, 3,5-octadiène-2-one (premier des 2 pics), nonanal, 2-nonénal, (E,E)-2,4-nonadiénal, acide hexanoïque) nous permet de distinguer les produits acceptables des produits non-acceptables sur le critère « peinture », par analyse des volatils du produit mis en suspension aqueuse.

### *Elaboration du modèle simplifié*

Afin de simplifier le modèle, il est décidé de retenir les composés ayant le plus grand impact sur l'odeur globale des compositions de biomasse de microalgues riches en protéines selon l'invention, c'est-à-dire les composés avec des seuils olfactifs extrêmement bas.

Ces valeurs d'arômes individuelles (= concentration du composé/son seuil olfactif) sont représentées dans le **Figure 5**.

Tenant compte de la concentration et du seuil olfactif de chaque composé, quatre composés semblent particulièrement importants pour les propriétés sensorielles des compositions de biomasse de microalgues riches en protéines selon l'invention : le 3,5-octadiène-2-ol (ou 3-octène-2-one, option ne faisant pas partie de l'invention) (floral-zeste), le 1-octène-3-ol (champignon-solvant, peinture, champignon-encre), le 3,5-octadiène-2-one (le premier des deux pics, florale), et le (E,E)-2,4-nonadiénal (huileuse-oxydée).

Les descripteurs individuels de ces quatre composés rejoignent fort bien l'odeur globale perçue des compositions de biomasse de microalgues riches en protéines selon l'invention inacceptables.

Il est donc possible d'établir une valeur d'arôme globale pour les compositions de biomasse de microalgues riches en protéines, basée sur ces quatre composés :

Valeur globale d'arôme = $\sum$ des valeurs arômes individuels du 3,5-octadiène-2-ol (ou 3-octène-2-one, option ne faisant pas partie de l'invention), du 1-octène-3-ol, du 3,5-octadiène-2-one, et du (E,E)-2,4-nonadiénal.

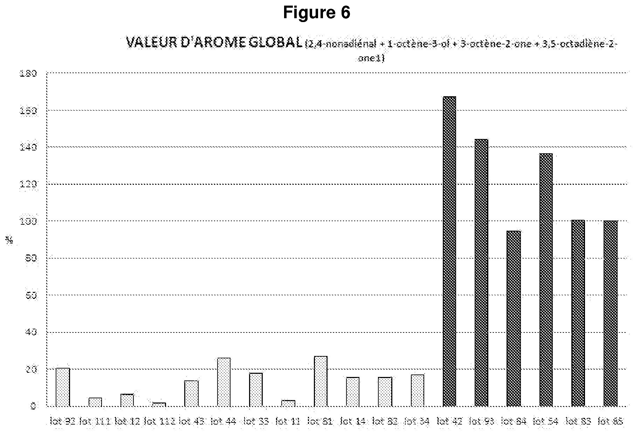

Comme le montre la **Figure 6**, il est désormais aisé de classer les différents lots de compositions de biomasse de microalgues riches en protéines en deux familles :

- les acceptables : il s'agit des lots 111, 92, 12, 112, 43, 33, 33, 81, 14, 44, 82 et 34 ;
- les inacceptables : il s'agit des lots 83, 84 et 85.

A noter que le lot 85, lot de qualité organoleptique inacceptable selon l'exemple 1, présente une valeur d'arôme de 100 %.

Les lots acceptables présentent donc bien une valeur d'arome globale comprise entre 0 et 40 % par rapport à celle de composition de farine de microalgues de référence organoleptique inacceptable, en l'occurrence ici le lot 85.

Les lots 42, 93 et 54 présentent une valeur globale d'arôme, sur la base des quatre composés organoleptiques, bien supérieure à celle du lot 85 de référence.

Cependant, il faut noter que le lot 85 a été défini inacceptable sur le seul descripteur "peinture".

Les lots 42, 93 et 54, sur le plan de l'analyse des composés organiques volatils, sont particulièrement marqués, sans doute par un effet de synergie entre composés organiques volatils.

Il n'en demeure pas moins que le modèle simplifié basé sur cette sélection des 4 composés organiques volatils sur les 11 de référence permet de classer les compositions de biomasses de microalgues riches en protéines en deux familles distinctes aisément identifiables.

### Description des Figures

FIGURE 1 : Note moyenne obtenue au descripteur peinture par chacune des compositions.

FIGURE 2 : Chromatogrammes (TIC) des composés organiques volatils prélevés dans les échantillons par SPME en suspension aqueuse

FIGURE 3 : Intégration de la totalité des pics pour la zone 3,2-35,0 min des chromatogrammes (SPME en suspension aqueuse)

FIGURE 4: Teneurs relatives en 11 composés sélectionnés prélevés dans l'espace des échantillons par SPME en suspension aqueuse FIGURE 5 : Valeurs d'arômes individuelles des 11 composés sélectionnés

FIGURE 6 : Valeur d'arôme globale basée sur 4 composés

**Revendications**

1. Méthode pour déterminer la qualité organoleptique d'une composition de biomasse de microalgues riche en protéines comprenant au minimum 50 % de protéines en poids sec de biomasse de microalgues, **caractérisée en ce que** la méthode comprend la détermination de la teneur en 11 composés organiques volatils, les 11 composés organiques volatils étant le pentanal, l'hexanal, le 1-octène-3-ol, le 2-pentylfurane, l'octanal, le 3,5-octadiène-2-ol (ou 3-octène-2-one), le 3,5-octadiène-2-one, le nonanal, le 2-nonénal, le (E,E)-2,4-nonadiénal et l'acide hexanoïque, la biomasse de microalgues comprenant plus de 50 % de protéines en poids sec de biomasse et les microalgues étant du genre Chlorella.

2. Méthode selon la revendication 1, dans laquelle la teneur en 11 composés organiques volatils est déterminée par SPME / GC, de préférence par SPME / GC-MS.

3. Méthode selon la revendication 1 ou 2, **caractérisée en ce que** la teneur en 11 composés organiques volatils est déterminée par la surface des pics de chromatographie après SPME / GC.

4. Méthode selon l'une quelconque des revendications 1-3, **caractérisée en ce que** la teneur en 11 composés organiques volatils, en particulier la surface des pics de chromatographie correspondant aux 11 composés organiques volatils, est comparée à celle de biomasse(s) de microalgues riche en protéines de référence pour laquelle ou lesquelles les qualités organoleptiques sont définies, notamment comme inacceptable ou acceptable.

5. Méthode pour déterminer la qualité organoleptique d'une composition de biomasse de microalgues riche en protéines comprenant au minimum 50 % de protéines en poids sec de biomasse de microalgues, **caractérisée en ce que** la méthode comprend la détermination de la teneur en 4 composés organiques volatils, ces 4 composés organiques étant le 3,5-octadiène-2-ol, le 1-octène-3-ol, du 3,5-octadiène-2-one, et le (E,E)-2,4-nonadiénal et **en ce que** l'on calcule une valeur d'arôme globale par la somme des valeurs d'arômes individuelles, lesdites valeurs d'arômes individuelles étant calculées en divisant la concentration du composé par son seuil olfactif, du 3,5-octadiène-2-ol, le 1-octène-3-ol, du 3,5-octadiène-2-one, et le (E,E)-2,4-nonadiénal,
la biomasse de microalgues comprenant plus de 50 % de protéines en poids sec de biomasse et les microalgues étant du genre Chlorella.

6. Méthode de sélection de compositions de biomasse de microalgues riches en protéines présentant un profil organoleptique acceptable, **caractérisée en ce que** la qualité organoleptique est déterminée par la méthode selon l'une quelconque des revendications 1 à 5 et que la composition est sélectionnée lorsque la valeur d'arôme globale calculée par la méthode selon la revendication 5 est comprise entre 0 et 40 % par rapport à celle d'une composition de biomasse de microalgues de référence organoleptique inacceptable, **caractérisée en ce que** la biomasse de microalgues comprend plus de 50 % de protéines en poids sec de biomasse et **en ce que** les microalgues sont du genre Chlorella.

7. Méthode selon l'une quelconque des revendications 1-5, **caractérisée en ce que** les microalgues sont choisies dans le groupe constitué de *Chlorella vulgaris, Chlorella sorokiniana* et *Chlorella protothecoides,* et plus particulièrement *Chlorella protothecoides.*


**Patentansprüche**

1. Verfahren zur Bestimmung der organoleptischen Qualität einer proteinreichen Mikroalgenbiomasse-Zusammensetzung, umfassend mindestens 50 Trockengew.-% Mikroalgenbiomasse-Proteine, **dadurch gekennzeichnet, dass** das Verfahren die Bestimmung des Gehalts von 11 flüchtigen organischen Verbindungen umfasst, wobei die 11 flüchtigen organischen Verbindungen Pentenal, Hexanal, 1-Octen-3-ol, 2-Pentylfuran, Octanal, 3,5-Octadien-2-ol (oder 3-Octen-2-on), 3,5-Octadien-2-on, Nonanal, 2-Nonenal, (E,E)-2,4-Nonadienal und Hexansäure sind, wobei die Mikroalgenbiomasse mehr als 50 Trockengew.-% Biomasse-Proteine umfasst und die Mikroalgen zur Gattung Chlorella gehören.

2. Verfahren gemäß Anspruch 1, wobei der Gehalt von 11 flüchtigen organischen Verbindungen mit SPME / GC, vorzugsweise mit SPME / GC-MS bestimmt wird.

3. Verfahren gemäß Anspruch 1 oder 2, **dadurch gekennzeichnet, dass** der Gehalt von 11 flüchtigen organischen Verbindungen mithilfe der Fläche der chromatographischen Peaks nach SPME / GC bestimmt wird.

4.  Verfahren gemäß einem der Ansprüche 1 bis 3, **dadurch gekennzeichnet, dass** der Gehalt von 11 flüchtigen organischen Verbindungen, insbesondere die Fläche der chromatographischen Peaks, die den 11 flüchtigen organischen Verbindungen entsprechen, mit derjenigen der proteinreichen Referenz-Mikroalgenbiomasse(n) verglichen wird, für die die organoleptischen Qualitäten, insbesondere als inakzeptabel oder akzeptabel definiert sind.

5.  Verfahren zur Bestimmung der organoleptischen Qualität einer proteinreichen Mikroalgenbiomasse-Zusammensetzung, umfassend mindestens 50 Trockengew.-% Mikroalgenbiomasse-Proteine, **dadurch gekennzeichnet, dass** das Verfahren die Bestimmung des Gehalts von 4 flüchtigen organischen Verbindungen umfasst, wobei diese 4 organischen Verbindungen 3,5-Octadien-2-ol, 1-Octen-3-ol, 3,5-Octadien-2-on und (E,E)-2,4-Nonadienal sind und dass man einen Gesamtaromawert mithilfe der Summe der einzelnen Aromawerte von 3,5 Octadien-2-ol, 1-Octen-3-ol, 3,5-Octadien-2-on und (E,E)-2,4-Nonadienal berechnet, wobei die einzelnen Aromawerte durch Dividieren der Konzentration der Verbindung durch ihre olfaktorische Schwelle berechnet werden, wobei die Mikroalgenbiomasse mehr als 50 Trockengew.-% Biomasse-Proteine umfasst und die Mikroalgen zur Gattung Chlorella gehören.

6.  Verfahren zur Auswahl von proteinreichen Mikroalgenbiomasse-Zusammensetzungen, die ein akzeptables organoleptisches Profil aufweisen, **dadurch gekennzeichnet, dass** die organoleptische Qualität mit dem Verfahren gemäß einem der Ansprüche 1 bis 5 bestimmt wird und dass die Zusammensetzung ausgewählt wird, wenn der mit dem Verfahren gemäß Anspruch 5 berechnete Gesamtaromawert zwischen 0 und 40% bezogen auf den Wert einer organoleptisch inakzeptablen Mikroalgenbiomasse-Referenzzusammensetzung beträgt, **dadurch gekennzeichnet, dass** die Mikroalgenbiomasse mehr als 50 Trockengew.-% Biomasse-Proteine umfasst und dass die Mikroalgen zur Gattung Chlorella gehören.

7.  Verfahren gemäß einem der Ansprüche 1 bis 5, **dadurch gekennzeichnet, dass** die Mikroalgen aus der Gruppe ausgewählt sind, bestehend aus *Chlorella vulgaris*, *Chlorella sorokiniana* und *Chlorella protothecoides*, und insbesondere *Chlorella protothecoides* sind.

**Claims**

1.  A method for determining the organoleptic quality of a protein-rich microalgal biomass composition comprising at least 50% protein by dry weight of microalgal biomass, **characterized in that** the method comprises determining the content of 11 volatile organic compounds, the 11 volatile organic compounds being pentanal, hexanal, 1-octen-3-ol, 2-pentylfuran, octanal, 3,5-octadien-2-ol (or 3-octen-2-one), 3,5-octadien-2-one, nonanal, 2-nonenal, (E,E)-2,4-nonadienal and hexanoic acid, the microalgal biomass comprising more than 50% protein by dry weight of biomass and the microalgae being of the genus *Chlorella.*

2.  The method as claimed in claim 1, wherein the content of 11 volatile organic compounds is determined by SPME / GC, preferably by SPME / GC-MS.

3.  The method as claimed in claim 1 or 2, **characterized in that** the content of 11 volatile organic compounds is determined by the area of the chromatographic peaks after SPME / GC.

4.  The method as claimed in any one of claims 1 to 3, **characterized in that** the content of 11 volatile organic compounds, in particular the area of the chromatographic peaks corresponding to the 11 volatile organic compounds, is compared with that of reference protein-rich microalgal biomass(es) for which the organoleptic qualities are defined, in particular as unacceptable or acceptable.

5.  A method for determining the organoleptic quality of a protein-rich microalgal biomass composition comprising at least 50% protein by dry weight of microalgal biomass, **characterized in that** the method comprises determining the content of 4 volatile organic compounds, these 4 volatile organic compounds being 3,5-octadien-2-ol, 1-octen-3-ol, 3,5-octadien-2-one and (E,E)-2,4-nonadienal, and **in that** a flavor value is calculated by the sum of the individual flavor values, said individual flavor values being calculated by dividing the concentration of the compound by its odor threshold, of 3,5-octadien-2-ol, 1-octene-3-ol, 3,5-octadien-2-one, and (E,E)-2,4-nonadienal, the microalgal biomass of comprising more than 50% protein by dry weight of biomass and the microalgae being of the genus *Chlorella.*

6. A method for selecting protein-rich microalgal biomass compositions having an acceptable organoleptic profile, **characterized in that** the organoleptic quality is determined by the method as claimed in any one of claims 1 to 5 and that the composition is selected when the overall flavor value calculated by the method as claimed in claim 5 is comprised between 0 and 40% relative to that of an organoleptically unacceptable reference microalgal biomass composition, **characterized in that** the biomass of microalgae comprises more than 50% protein by dry weight biomass and **in that** the microalgae are of the genus *Chlorella*.

7. The method as claimed in any one of claims 1 to 5, **characterized in that** the microalgae are selected from the group consisting of *Chlorella vulgaris*, *Chlorella sorokiniana* and *Chlorella protothecoides*, and more particularly *Chlorella protothecoides*.

Figure 1

# Figure 2

# Figure 2 (suite)

# Figure 3

**Figure 4**

**Figure 5**

# Figure 6

VALEUR D'AROME GLOBAL (2,4-nonadiénal + 1-octène-3-ol + 3-octène-2-one + 3,5-octadiène-2-one1)

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010045368 A
- EP 2948001 A0

**Littérature non-brevet citée dans la description**

- **DE BECKER.** *Biotechnology Advances,* 2007, vol. 25, 207-210
- **FRADIQUE.** *J Sci Food Agric,* 2010, vol. 90, 1656-1664
- **QUANT À SUN.** *J Appl Phycol,* 2012, vol. 24, 1003-1013
- **H. JELEN.** *Journal of Chromatographic Science,* Août 2006, vol. 44